(19) Europäisches Patentamt
European Patent Office
Office européen des brevets

(11) Publication number: **0 470 275 B1**

(12) # EUROPEAN PATENT SPECIFICATION

(45) Date of publication of patent specification: **13.07.94**

(51) Int. Cl.5: **A61K 7/48**

(21) Application number: **90115090.4**

(22) Date of filing: **06.08.90**

(54) **Skin whitening cosmetics.**

(43) Date of publication of application:
**12.02.92 Bulletin 92/07**

(45) Publication of the grant of the patent:
**13.07.94 Bulletin 94/28**

(84) Designated Contracting States:
**AT BE CH DE DK ES FR GB GR IT LI LU NL SE**

(56) References cited:
**EP-A- 0 160 430**
**EP-A- 0 197 485**
**FR-A- 2 604 624**

**PATENT ABSTRACTS OF JAPAN, vol. 13, no. 107 (C-576)[3455], 14th March 1989; & JP-A-63 284 109 (SUNSTAR INC.) 21-11-1988**

(73) Proprietor: **SUNSTAR KABUSHIKI KAISHA**
**3-1, Asahi-machi**
**Takatsuki-shi Osaka 569(JP)**

(72) Inventor: **Shimizu, Mitsuaki**
**14-2, Nishinokyo Kitaenmachi,**
**Nakagyo-ku**
**Kyoto-shi, Kyoto(JP)**
Inventor: **Kato, Hisatoyo**
**10-1, Kamihamuro 2-chome**
**Takatsuki-shi, Osaka(JP)**
Inventor: **Ozasa, Yoshitsugu**
**37-12, Hieidaira 2-chome**
**Otsu-shi, Shiga(JP)**
Inventor: **Ando, Hideya**
**2-3-402, Yokoo 9-chome**
**Suma-ku, Kobe-shi, Hyogo(JP)**
Inventor: **Hashimoto, Akira**
**14-86-307, Makita-cho**
**Takatsuki-shi, Osaka(JP)**

(74) Representative: **Patentanwälte Grünecker, Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 470 275 B1

## Description

FIELD OF THE INVENTION

This invention relates to a skin whitening cosmetic containing a specific fatty acid or a derivative thereof which is effective to eliminate or prevent skin browning or pigmentation in the skin, e.g., spots or freckles, due to ultraviolet light.

BACKGROUND OF THE INVENTION

Known cosmetics for giving fairness to the facial skin include compositions containing ascorbic acid or a derivative thereof or a tyrosinase inhibitor, e.g., a placenta extract and kojic acid or a derivative thereof. Although these conventional skin whitening cosmetics exhibit inhibitory activity against melanin production when tested in vitro using tissue cultures, they have not succeeded in obtaining sufficient effects on elimination of pigmentation when actually applied to the skin.

As a result of extensive researches on various components for their skin whitening effects, the inventors have previously discovered that incorporation of a specific fatty acid or a salt or an ester thereof into cosmetics brings about excellent skin whitening effects and filed an application for patent. It turned out, however, that such a fatty acid or a derivative thereof is still insufficient in skin whitening effect upon actual use when combined with fats and oils generally employed in conventional cosmetics.

SUMMARY OF THE INVENTION

Accordingly, an object of this invention is to provide a cosmetic containing a specific fatty acid or a salt or an ester thereof in which the skin whitening effects of the fatty acid or a salt or an ester thereof can be fully manifested.

Other objects and effects of the present invention will be apparent from the following description.

The present inventors have conducted extensive investigations and, as a result, found that a combination of a specific fatty acid, a salt thereof or an ester thereof with a mono- or dihydric alcohol, and a silicone oil unexpectedly produces excellent effects on elimination or prevention of pigmentation in the skin, and thus reached the present invention.

That is, the present invention relates to a skin whitening cosmetic comprising (a) at least one compound selected from the group consisting of a free fatty acid having from 18 to 22 carbon atoms and containing at least two unsaturated bonds per molecule, a salt of said fatty acid, and an ester of said fatty acid with a monohydric or dihydric alcohol, and (b) at least one silicone oil selected from the group consisting of compounds represented by formulae (I), (II), and (III):

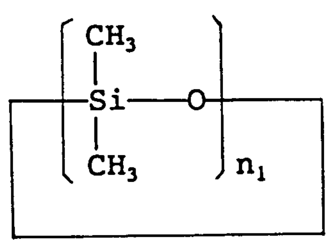

(I)

wherein $n_1$ represents an integer of from 4 to 6.

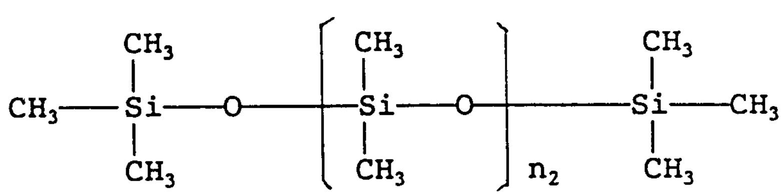

(II)

wherein $n_2$ represents an integer of from 1 to 400.

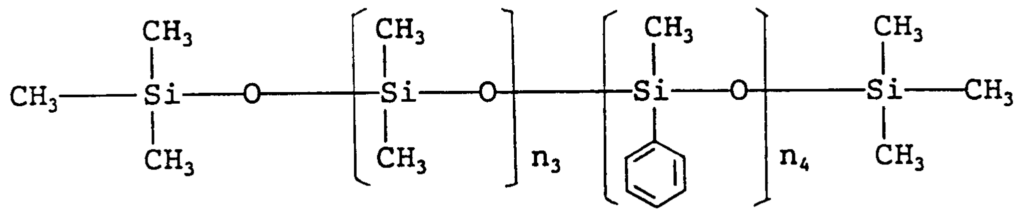

(III)

wherein $n_3$ and $n_4$ each represents an integer, the sum of $n_3$ and $n_4$ being from 1 to 400.

The present invention further relates to a skin whitening cosmetic comprising (c) ethanol in addition to the above-described components (a) and (b).

## DETAILED DESCRIPTION OF THE INVENTION

Fatty acids having from 18 to 22 carbon atoms and at least two unsaturated bonds per molecule, e.g., linoleic acid and γ-linolenic acid, which can be used in the present invention are contained in vegetable and animal fats and oils. Rarely are these fatty acids present in a free form, and most of them are present in the form of triglyceride. Such triglycerides reveal no such significant effects on inhibition of pigmentation in animal experiments as are observed with free fatty acids or alkyl esters thereof. Neither do saturated fatty acids, e.g., palmitic acid and stearic acid, and in some cases, saturated fatty acids rather accelerate melamine production. Considering that these saturated fatty acids are present in vegetable and animal fats and oils in large quantities, it is preferable to use a purified unsaturated fatty acid in the preparation of the cosmetic of the present invention.

Examples of the free unsaturated fatty acids containing from 18 to 22 carbon atoms and at least two unsaturated bonds per molecule include linoleic acid, linoelaidic acid, α-linolenic acid, γ-linolenic acid, dihomo-γ-linolenic acid, arachidonic acid, eicosapentaenoic acid, and docosahexaenoic acid. These fatty acids may be used either individually or in combination of two or more thereof.

These free unsaturated fatty acids are obtained by saponification of natural fats and oils. Examples of the natural fats and oils include vegetable fats and oils, e.g., linseed oil, safflower oil, sunflower oil, soybean oil, rape oil, corn oil, sesame oil, and grape seed oil; and marine animal oils, e.g., cuttlefish oil, sardine oil, saury oil, and cod liver oil.

Examples of salts of these free fatty acids which can be used in the present invention include metal salts, e.g., a sodium salt and a potassium salt; amino acid salts, e.g., an arginine salt and a lysine salt; and amine salts, e.g., a triethanolamine salt and a monoethanolamine salt.

Examples of alkyl esters of the fatty acids which can be used in the present invention include esters with monohydric alcohols, e.g., methanol, ethanol, and isopropyl alcohol; and dihydric alcohols, e.g., ethylene glycol, propylene glycol, and 1,3-butylene glycol.

Among the above free unsaturated fatty acids and a salt or an ester of these fatty acids, linoleic acid, α-linolenic acid, γ-linolenic acid, arachidonic acid, and sodium linoleate are preferably used in the present invention. Particularly, linoleic acid and α-linolenic acid are more preferred.

The free fatty acid and a salt or an ester thereof are preferably used in an amount of from 0.1 to 10% by weight, and preferably from 0.1 to 5% by weight, based on the total amount of the cosmetic of the present invention. If the amount is less than 0.1% by weight, no inhibitory activity on pigmentation tends to be exerted. If it exceeds 10% by weight, skin irritation may be caused.

The silicone oil which is used in combination with the above-described component (a) is selected from the compounds represented by the above-described formulae (I), (II), and (III).

Examples of the silicone oil of formula (I) include KF994 and KF995 (products of Shin-Etsu Chemical Co., Ltd.) and SH244 and SH245 (products of Toray Silicone Co., Ltd.). Examples of the silicone oil of formula (II) include KF96 1 cs and KF96 2 cs (products of Shin-Etsu Chemical Co., Ltd.) and SH200c/1 cs, SH200c/10 cs, SH200c/100 cs, and SH200c/500 cs (products of Toray Silicone Co., Ltd.). Examples of the silicone oil of formula (III) include SH556 (product of Toray Silicone Co., Ltd.).

Among the above silicone oils, compounds represented by formula (I) in which $n_1$ represents an integer of from 4 to 6 and compounds represented by formula (II) in which $n_2$ represents an integer of from 1 to 10 are preferably used in the present invention. Particularly, compounds represented by formula (I) in which $n_1$ represents an integer of from 4 to 5 and compounds represented by formula (II) in which $n_2$ represents an

integer of from 1 to 7 are more preferred.

The silicone oil is preferably used in an amount of 1% by weight or more, more preferably 10% by weight or more, and particularly preferably 50% by weight or more, based on the total amount of the cosmetic of the present invention.

Silicone oil to be used can be arbitrarily chosen from the above-described compounds. Two or more of these silicone oils can be used in combination for the purpose of improving feel on application, solubility of fatty acids, and anti-freezing properties at low temperatures.

For example: although octamethylcyclotetrasiloxane freezes at low temperature (10 °C or lower), freezing at low temperatures can be prevented by adding decamethylcyclopentasiloxane, methylphenylsiloxane or dimethylsiloxane; although linoleic acid, α-linolenic acid and the like are not soluble in dimethylpolysiloxane, the solubilities can be improved by adding methylphenylsiloxane or octamethylcyclotetrasilxane; and although octamethylcyclotetrasilxane and decamethylcyclopentasiloxane have less oleophilic feel upon use and therefore are not excellent in feel of cosmetics on application, the feel of cosmetics can be improved by adding dimethylsiloxane or methylphenylsiloxane.

For the same purpose, the cosmetic of the present invention may further contain ethanol. Ethanol is preferably used in an amount not more than 50.0% by weight, more preferably from 1 to 40% by weight, and particularly preferably from 3 to 25% by weight, based on the total amount of the cosmetic. If the amount of ethanol exceeds 50.0% by weight, the cosmetic not only causes irritation to the skin but feels cold on application due to volatilization of ethanol, thus greatly deteriorating feel on use.

The cosmetics of the present invention can be formulated into cosmetic oils, creams, and so on according to known techniques.

If desired, the cosmetics of the present invention may further contain other components generally employed in cosmetics as long as the effects of the present invention are not impaired.

The conventional compositions and its production process as well as the conventional components added therefor of the cosmetic compositions are described, e.g., in Keshohin-Gaku (Cosmetic Science), edited by T. Ikeda, published on May 20, 1979 by Nanzando, Japan, which is incorporated herein by reference, but the present invention is not construed as being limited thereto.

Furthermore, if desired, the cosmetics of the present invention can contain various conventional additives, such as melanin production inhibitors (whitening agents) (e.g., vitamin C and placenta extract), anti-inflammatory agents, and antioxidants, as long as the effects of the present invention are not impaired.

Examples of the conventional additives and their addition amounts are shown in Table 1 below but they are not limited thereto. The addition amounts are shown in terms of percent by weight (except Vitamin A) based on the total amount of the cosmetic compositions.

TABLE 1

| Additives | Addition amount (% by weight) |
|---|---|
| Whitening agent | |
| Placenta extract<br>Kojic acid<br>Photosensitive element No. 201<br>Plant extract<br>Vitamin A | 0.1 to 3<br>0.1 to 3<br>0.0001 to 0.002<br>0.01 to 1<br>500 IU to 2,500 IU |
| Anti-inflammatory agent | |
| Dipotassium grycyrrhizic acid<br>Stearil grycyrrethinate<br>Allantoin<br>$\epsilon$-Aminocaproic acid<br>Methyl salicylate | 0.01 to 0.2<br>0.01 to 0.2<br>0.01 to 0.2<br>0.01 to 0.1<br>0.01 to 0.1 |
| Antioxidant | |
| Dibutylhydroxytoluene<br>Butylhydroxyanisole<br>Propyl gallate<br>Tocopherol<br>Erythorbic acid | 0.01 to 1<br>0.01 to 1<br>0.01 to 0.2<br>0.01 to 1<br>0.0001 to 0.05 |

The cosmetics according to the present invention can be applied to the skin by conventional manners. For example, a lotion containing 1 wt% of the compound of the present invention can be applied by hands 1 to several times per day; one or two drops of a cosmetic oil containing 3 wt% of the compounds can be applied to the portion at which pigmentation occurs by fingers 1 to 3 times per day; a cream containing 5 wt% of the compound can be applied by hands 1 to several times per day; an emulsion containing 2 wt% of the compound can be applied by hands 1 to 3 times per day; and a pack can be used in such a manner that 5 to 10 g of the pack is applied to the facial skin other than eyes and nose, and it allows to stand for about 30 minutes followed by being removed 1 to 2 times per week.

Skin whitening effects according to the present invention was tested as follows.

Test Method:

Ultraviolet light (UVB intensity: 1 J/cm$^2$) was 4 times (twice per week) irradiated on the shaved back of English brown guinea pigs. One week after from the irradiation, a pigmentation was formed.

On 6 cm$^2$ (2 cm x 3 cm) of the back of the guinea pigs having the pigmentation, 0.05 ml of a solution of a linoleic acid in each of bases shown in Table 1 below was 20 times (once per day) applied.

The degree of pigmentation was observed with eyes and evaluated as follows taking that of the area where no test compound was applied as a standard (0).

0: No reduction in pigmentation was observed.
-1: Slight reduction in pigmentation was observed.
-2: Medium reduction in pigmentation was observed.
-3: Considerable reduction in pigmentation was observed.

The results of the evaluation are shown in Table 2 below.

**TABLE 2**

| Base | Conc. of Linoleic Acid (wt%) | Evaluation |
|---|---|---|
| White petrolatum | 5.0 | 0 |
| Liquid paraffin | 5.0 | -1 |
| " | 0.1 | 0 |
| Squalane | 5.0 | -1 |
| Isocetyl stearate | 5.0 | -1 |
| Octamethylcyclotetrasiloxane*1 | 0.1 | -2 |
| " | 5.0 | -3 |
| Decamethylcyclopentasiloxane*2 | 0.1 | -2 |
| " | 5.0 | -3 |
| Dimethylpolysiloxane (500 cs) | 0.1 | -2 |
| " (100 cs) | 0.1 | -2 |
| " (10 cs) | 0.1 | -2 |
| " (10 cs) | 5.0 | -3 |
| " (1 cs) | 0.1 | -2 |
| " (1 cs) | 5.0 | -3 |
| Methylphenylpolysiloxane | 0.1 | -2 |
| " | 5.0 | -3 |
| Dimethylpolysiloxane (500 cs)/methylphenylpolysiloxane (1/3 by weight) | 5.0 | -3 |
| Dimethylpolysiloxane (100 cs)/decamethylcyclopentasiloxane (1/1 by weight) | 5.0 | -3 |
| Octamethylcyclotetrasiloxane/dimethylpolysiloxane (500 cs) (8/12 by weight) | 0.1 | -2 |

Note: *1 Formula (I) (n=4)
*2 Formula (I) (n=5)

As is apparent from the results in Table 2, significant reduction of pigmentation can be obtained when linoleic acid is combined with a silicone oil or a mixture thereof as a base. It is also seen that the skin whitening effect increases with an increase in concentration of linoleic acid. Satisfactory effects can be obtained with linoleic acid of from 0.1 to 10% by weight, and preferably from 0.1 to 5% by weight. It was noted, however, that linoleic acid when used at high concentrations is not dissolved in a silicone oil base depending on the kind of the silicone oil.

Solubility of linoleic acid in a silicone oil or a mixed silicone oil is shown in Tables 3 and 4.

TABLE 3

| Kind of Silicone Oil | Conc. of Linoleic Acid 1% by wt. | 5% by wt. |
|---|---|---|
| Octamethylcyclotetrasiloxane | soluble | soluble |
| Decamethylcyclopentasiloxane | " | " |
| Dimethylpolysiloxane (500 cs) | " | insoluble |
| " (100 cs) | " | " |
| " (10 cs) | " | soluble |
| " (1 cs) | " | " |
| Methylphenylpolysiloxane | " | " |

TABLE 4
(Conc. of Linoleic Acid: 5% by wt.)

| Silicone Oil A: | A/B Weight Ratio | Silicone Oil B: Octamethyl-cyclotetra-siloxane | Decamethyl-cyclopenta-siloxane | Dimethyl-polysil-oxane (10 cs) | Dimethyl-polysil-oxane (2 cs) | Methylphenyl-polysiloxane |
|---|---|---|---|---|---|---|
| Dimethylpoly-siloxane (500 cs) | 3/1 | insoluble | insoluble | insoluble | insoluble | suspended |
| | 1/1 | soluble | soluble | " | " | soluble |
| | 1/3 | " | " | suspended | suspended | " |
| Dimethylpoly-siloxane (100 cs) | 3/1 | insoluble | insoluble | insoluble | insoluble | " |
| | 1/1 | soluble | soluble | " | " | " |
| | 1/3 | " | " | suspended | suspended | " |

TABLE 5
(Conc. of Linoleic Acid: 5% by weight)

| Silicone Oil C: | C/D Weight Ratio | Silicone Oil D (freezing point: 0°C or less) | | | | | |
|---|---|---|---|---|---|---|---|
| | | Decamethylcyclopentasiloxane | Dimethylpolysiloxane (500 cs) | Dimethylpolysiloxane (100 cs) | Dimethylpolysiloxane (10 cs) | Dimethylpolysiloxane (1 cs) | Methylphenylpolysiloxane |
| Octamethylcyclotetrasiloxane (freezing point: 16°C) | 17/3 | X | X | X | X | X | X |
| " | 14/6 | X | X | X | X | X | X |
| " | 12/8 | O | X | X | O | O | O |
| " | 10/10 | O | X | X | O | O | O |
| " | 8/12 | O | O | O | O | O | O |
| " | 6/14 | O | O | O | O | O | O |
| " | 4/16 | O | O | O | O | O | O |
| " | 2/18 | O | O | O | O | O | O |

TABLE 6

| Octamethylcyclo-tetrasiloxane (% by wt.) | Ethanol (% by wt.) | Linoleic Acid (% by wt.) | Evaluation |
|---|---|---|---|
| 93 | 2 | 5 | X |
| 91 | 4 | 5 | X |
| 89 | 6 | 5 | X |
| 87 | 8 | 5 | O |
| 85 | 10 | 5 | O |

Note: O: Not freese at 0°C

X: Freeze at 0°C

As is apparent from the results in Table 3, linoleic acid of high concentration lacks solubility in dimethylpolysiloxane having a viscosity of 100 cs or more. In the case, solubility of linoleic acid can be increased by adding an adequate amount of octamethylcyclotetrasiloxane, decamethylcyclopentasiloxane, methylphenylpolysiloxane, etc. as shown in Table 4.

Tables 5 and 6 shows low temperature stability of octamethylcyclotetrasiloxane. Although octamethylcyclotetrasiloxane freezes at low temperatures because of its high coagulation point, freezing can be prevented by a combined use of an adequate amount of decamethylcyclopentasiloxane, dimethylpolysiloxane, methylphenylpolysiloxane, or ethanol.

The present invention is now illustrated in greater detail by way of the following Examples. All the percents are by weight unless otherwise indicated.

## EXAMPLE 1

Cosmetic Oil:

| | |
|---|---|
| Linoleic acid | 1.5% |
| Octamethylcyclotetrasiloxane | 78.3% |
| Decamethylcyclopentasiloxane | 20.0% |
| Dibutylhydroxytoluene | 0.1% |
| Tocopherol | 0.1% |
| Total: | 100.0% |

## EXAMPLE 2

Cosmetic Oil:

| | |
|---|---|
| Eicosapentaenoic acid | 2.0% |
| Dimethylpolysiloxane (2 cs) | 89.8% |
| Retinol | 0.1% |
| Ethyl p-dimethylaminobenzoate | 2.0% |
| Propyl p-hydroxybenzoate | 0.1% |
| Methylphenylpolysiloxane | 6.0% |
| Total: | 100.0% |

## EXAMPLE 3

Cosmetic Oil:

| | |
|---|---|
| γ-Linolenic acid | 1.5% |
| Octamethylcyclotetrasiloxane | 79.6% |
| Dimethylpolysiloxane (2 cs) | 17.8% |
| Soybean phospholipid | 0.1% |
| Cinoxate | 1.0% |
| | Total: 100.0% |

## EXAMPLE 4

Cosmetic Oil:

| | |
|---|---|
| Ethyl linoleate | 1.5% |
| Octamethylcyclotetrasiloxane | 60.0% |
| Decamethylcyclopentasiloxane | 20.0% |
| Dimethylpolysiloxane (100 cs) | 18.3% |
| Butylhydroxyanisole | 0.2% |
| | Total: 100.0% |

## EXAMPLE 5

Cosmetic Oil:

| | |
|---|---|
| γ-Linolenic acid | 2.0% |
| Octamethylcyclotetrasiloxane | 85.0% |
| Dimethylpolysiloxane (50 cs) | 12.8% |
| Tocopherol | 0.2% |
| | Total: 100.0% |

## EXAMPLE 6

Cosmetic Oil:

| | |
|---|---|
| α-Linoleic acid | 2.5% |
| Dimethylpolysiloxane (2 cs) | 35.0% |
| Octamethylcyclotetrasiloxane | 42.2% |
| Ethanol | 20.0% |
| Stearyl glycyrrhetinate | 0.1% |
| Ascorbyl stearate | 0.1% |
| Dibutylhydroxytoluene | 0.1% |
| Total: | 100.0% |

In the above Examples 1 to 6, all the component were uniformly mixed and dissolved with each other to obtain cosmetic oils.

## EXAMPLE 7

Cream:

| | |
|---|---|
| Linoleic acid | 1.0% |
| Dibutylhydroxytoluene | 0.05% |
| Decamethylcyclopentasiloxane | 15.0% |
| Dimethylpolysiloxane (500 cs) | 2.0% |
| Monoglyceryl linoleate | 2.0% |
| Monoglyceryl oleate | 2.0% |
| Olive oil | 2.0% |
| Methyl p-hydroxybenzoate | 0.1% |
| Citric acid | 0.05% |
| Concentrated glycerin | 15.0% |
| Dimethylpolysiloxane/polyethylene glycol copolymer | 5.0% |
| Purified water | 55.8% |
| Total: | 100.0% |

In the above Examples 1 to 6, all the component were uniformly mixed and dissolved with each other to obtain a cream.

The cosmetics according to the present invention, when applied to the skin, eliminate or prevent browning or pigmentation of the skin due to ultraviolet light and exhibit excellent skin whitening effects.

## Claims

1. A skin whitening cosmetic comprising (a) at least one compound selected from the group consisting of a free fatty acid having from 18 to 22 carbon atoms and containing at least two unsaturated bonds per molecule, a salt of said fatty acid, and an ester of said fatty acid with a monohydric or dihydric alcohol, and (b) at least one silicone oil selected from the group consisting of compounds represented by formulae (I), (II), and (III):

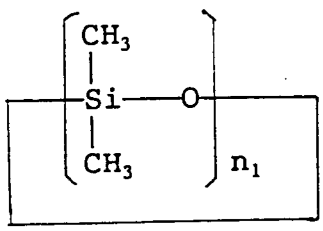

(I)

wherein $n_1$ represents an integer of from 4 to 6,

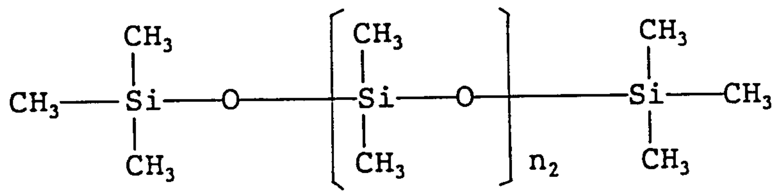

(II)

wherein $n_2$ represents an integer of from 1 to 400,

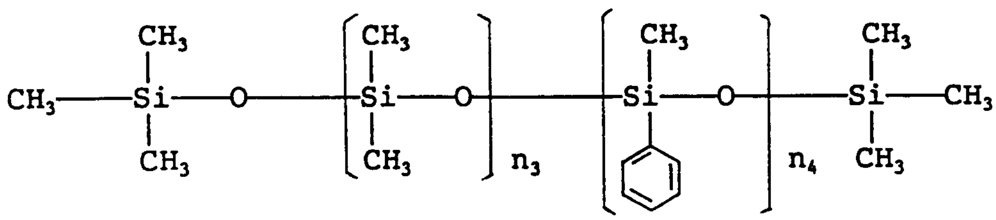

(III)

wherein $n_3$ and $n_4$ each represents an integer, the sum of $n_3$ and $n_4$ being from 1 to 400.

2. A skin whitening cosmetic as claimed in claim 1, wherein said compound or compounds selected from the group consisting of a free fatty acid having from 18 to 22 carbon atoms and containing at least two unsaturated bonds per molecule, a salt of said fatty acid, and an ester of said fatty acid is/are present in an amount of from 0.1 to 10% by weight based on the weight of the cosmetic.

3. A skin whitening cosmetic as claimed in claim 1, wherein said cosmetic further comprises ethanol.

4. A skin whitening cosmetic as claimed in claim 3, wherein said ethanol is present in an amount not more than 50.0% by weight based on the weight of the cosmetic.

## Patentansprüche

1. Hautbleichendes Kosmetikum umfassend (a) mindestens eine Verbindung ausgewählt aus der Gruppe bestehend aus einer freien Fettsäure mit 18 bis 22 Kohlenstoffatomen, die mindestens zwei ungesättigte Bindungen pro Molekül enthält, einem Salz dieser Fettsäure und einem Ester dieser Fettsäure mit einem einwertigen oder Zweiwertigen Alkohol, und (b) mindestens ein Siliconöl ausgewählt aus der

Gruppe bestehend aus Verbindungen, die durch die Formeln (I), (II) und (III) dargestellt sind:

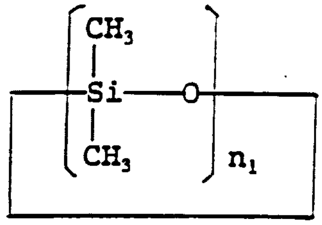

(I)

worin $n_1$ eine ganze Zahl von 4 bis 6 bedeutet,

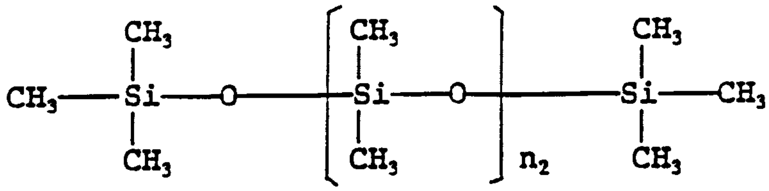

(II)

worin $n_2$ eine ganze Zahl von 1 bis 400 bedeutet,

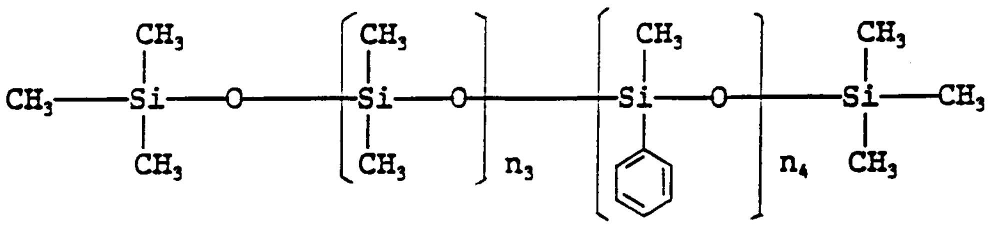

(III)

worin $n_3$ und $n_4$ jeweils eine ganze Zahl bedeutet, wobei die Summe von $n_3$ und $n_4$ 1 bis 400 beträgt.

**2.** Hautbleichendes Kosmetikum nach Anspruch 1, worin die Verbindung oder Verbindungen, welche ausgewählt sind aus der Gruppe bestehend aus einer freien Fettsäure mit 18 bis 22 Kohlenstoffatomen, die mindestens zwei ungesättigte Bindungen pro Molekül enthält, einem Salz dieser Fettsäure, und einem Ester dieser Fettsäure, in einer Menge von 0,1 bis 10 Gewichtsprozent, bezogen auf das Gewicht des Kosmetikums, vorhanden ist/sind.

**3.** Hautbleichendes Kosmetikum nach Anspruch 1, worin das Kosmetikum ferner Ethanol umfaßt.

**4.** Hautbleichendes Kosmetikum nach Anspruch 3, worin das Ethanol in einer Menge von nicht mehr als 50,0 Gewichtsprozent, bezogen auf das Gewicht des Kosmetikums, vorhanden ist.

**Revendications**

**1.** Un produit cosmétique pour le blanchiment de la peau comprenant (a) au moins un composé sélectionné dans le groupe constitué par un acide gras libre ayant entre 18 et 22 atomes de carbone et contenant au moins deux liaisons insaturées par molécule, par un sel dudit acide gras, et par un ester dudit acide gras avec un monoalcool ou un dialcool, et (b) au moins une huile de silicone sélectionnée dans le groupe constitué par les composés représentés par les formules (I), (II) et (III) :

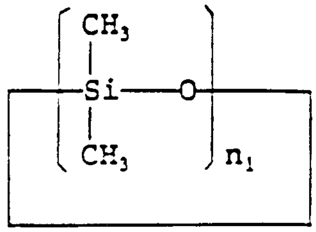

(I)

dans laquelle $n_1$ représente un entier compris entre 4 et 6,

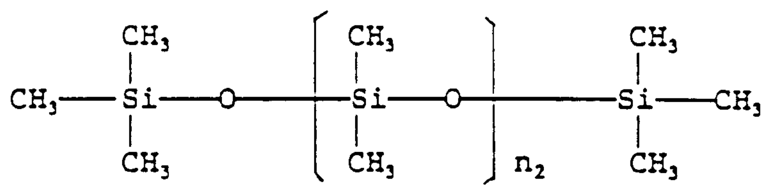

(II)

dans laquelle $n_2$ représente un entier compris entre 1 et 400,

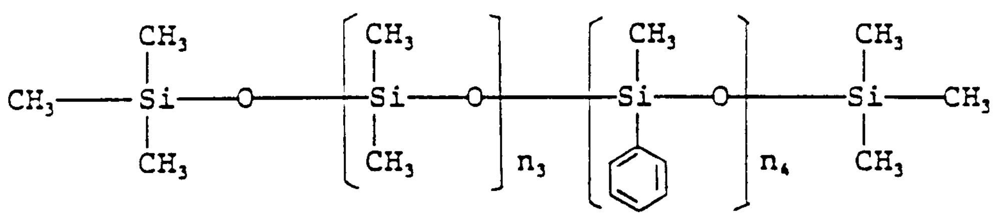

(III)

dans laquelle $n_3$ et $n_4$ représentent chacun un entier, la somme de $n_3$ et $n_4$ étant comprise entre 1 et 400.

2. Un produit cosmétique pour le blanchiment de la peau, comme revendiqué dans la revendication 1, dans lequel ledit composé ou lesdits composés, sélectionnés dans le groupe constitué par un acide gras libre ayant entre 18 et 22 atomes de carbone et contenant au moins deux liaisons insaturées par molécule, par un sel dudit acide gras, et par un ester dudit acide gras est/sont présent(s) dans une quantité comprise entre 0,1 et 10 % en poids, rapporté au poids du produit cosmétique.

3. Un produit cosmétique pour le blanchiment de la peau, comme revendiqué dans la revendication 1, dans lequel ledit produit cosmétique comprend en plus de l'éthanol.

4. Un produit cosmétique pour le blanchiment de la peau, comme revendiqué dans la revendication 3, dans lequel ledit éthanol est présent dans une quantité inférieure ou égale à 50 % en poids, rapporté au poids du produit cosmétique.